Europäisches Patentamt

European Patent Office

Office européen des brevets

(11) Publication number: **0 235 113**
A2

# EUROPEAN PATENT APPLICATION

(21) Application number: 87870027.7

(22) Date of filing: 26.02.87

(51) Int. Cl.³: **C 12 N 15/00**
C 12 N 5/00, C 12 P 21/00

(30) Priority: 28.02.86 US 835089
30.01.87 US 9420

(43) Date of publication of application:
02.09.87 Bulletin 87/36

(84) Designated Contracting States:
AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

(72) Inventor: Henri, Edward Mark
8224 Cadwalader Avenue
Elkins Park Pennsylvania 19117(US)

(72) Inventor: Jonak, Zdenka Ludmila
128 Ladderback Lane
Devon Pennsylvania 19333(US)

(72) Inventor: Machy, Patrick Simon Jacques
11 Rue Joinville
Provence 4 F-13600 La Ciotat(FR)

(74) Representative: Tasset, Gérard
SMITHKLINE - RIT rue de l'Institut, 89
B-1330 Rixensart(BE)

(54) Immortalization of primary cells.

(57) The use of electroporation as a transfection method to immortalize primary cells with oncogenic DNA/oncogene(s).

EP 0 235 113 A2

Croydon Printing Company Ltd.

SKB 14319

## TITLE

Immortalization of Primary Cells

## BACKGROUND OF THE INVENTION

## CROSS REFERENCE TO RELATED APPLICATIONS

This application is a continuation-in-part of Serial Number 835,089, filed February 28, 1986, which is pending.

This invention relates to a method of producing immortalized primary cells by transfection of such cells with oncogenic DNA/oncogene(s) using electroporation as a technique to deliver such DNA to these cells.

Transfection of DNA into mammalian cells has made an important contribution in the investigation of gene expression and regulation. This technique has helped in the discovery of new genes for which molecular probes are not available. The various methods used to introduce genes into cells can be divided into two systems, natural and artificial.

"Natural" transfection is accomplished by the infection of cells with intact (or genetically manipulated) viruses. Genetic information from viruses (DNA, RNA) can

be integrated into the host cell DNA, resulting in transformation of the host cell.

"Artificial" in vitro manipulation of DNA and its introduction into intact cells of different origin is usually accomplished by either chemical or manual methods. Several techniques have been developed to introduce DNA into somatic cells. These techniques can be divided into five types, depending on the way the macromolecules are delivered to the recipient cells:

1.  Microinjection [Graessmann et al., Hoppe-Seyler's Z Physiol. Chem., 352, 527-532, (1971); Capecchi, Cell, 22, 470-488 (1980)].

2.  Vehicle mediated transfer

    -   Erythrocyte ghosts [Furusawa et al., Methods in Cell Biol., 14, 17-80, (1976)]
    -   Liposomes [Poste et al., Methods in Cell Biol., 14, 33-71, (1976); Gregoriadis et al., Nature, 244, 170-172, (1973)]
    -   Reconstituted Sendai virus envelopes [Loyter et al., Exp. Cell Res., 139, 223-234 (1983)]

3.  Transfer aided by facilitators

    -   Calcium phosphate [Graham et al., Virology, 52, 456-467, (1973)]
    -   Calcium phosphate/polyethylene glycol-DMSO shock [Jonak et al., "Monoclonal Antibodies and Functional Cell Lines (Progress and Applications)," Plerum Press, 418-422, (1984)]

- DEAE-dextran [Graham, Adv. Cancer Res., 25, 1-51, (1977)]
- Polyethylene glycol/sucrose shock [Chu et al., Gene, 13, 197-202, (1981)]

4. Laser microbeam [Tsukakoshi et al., Applied Physics B., 35, 2284-2289, (1984)]

5. Electroporation [Neumann et al., Embo J., 1, 841-845, (1982); Potter et al., Proc. Natl. Acad. Sci., USA, 81, 7161-7165, (1984)]

These techniques have been used for introducing DNA, RNA, proteins, lipids, drugs, small molecules as well as organelles and virus particles into somatic cells. There are limitations in each one of these techniques and certain biological problems are best approached by using a particular method.

. DNA-mediated gene transfer is usually accomplished by various modifications of a calcium phosphate-DNA precipitation technique. This method requires formation of a DNA calcium phosphate-coprecipitate (45 minutes) and a time-consuming incubation (2 hours) of recipient cells with the coprecipitate. The precipitate is then taken up via an endocytotic-like process [Loyter et al., Ciba Found. Symp., 103, 163-175 (1982)]. This technique necessitates the use of specific cells of fibroblastic origin with endocytotic potential, thus limiting its application.

Various cell types have specific efficiencies with which they are capable of being transfected. Despite these differences, a variety of cell types have been successfully used for transfection. These include normal cells like lymphocytes, which were transfected in suspension by a calcium phosphate DNA-coprecipitation/polyethylene

- 4 -

0235113

glycol-DMSO technique [Jonak et al., **Hybridoma**, **3**, 107-118 (1984)].

Previous experiments (Jonak et al., **Hybridoma**, cited above), have demonstrated that the introduction of DNA isolated from human leukemia cells, into stimulated primary mouse lymphocytes by calcium phosphate DNA-coprecipitation/ polyethylene glycol-DMSO mediated delivery can produce cell lines with the capacity to proliferate continuously in culture. Therefore, the transfection of normal, stimulated lymphocytes with oncogenic DNA by such delivery technique was shown to be a new methodology to immortalize mouse lymphocytes and maintain these cells continuously in culture. Such new cell lines (immortalized mouse lymphocytes) will to produce their products in monoclonal form.

Potter et al., cited above, and Celis, **Biochem. J.**, **223**, 281-291, (1984), studied expression of cloned human and mouse Igk genes which were introduced into mouse pre-B cells and fibroblast cells by electroporation, and disclose that such method is applicable to many cell types, including those that are refractory to traditional transfection procedures.

Van Brunt, **Biotechnology**, **3**, 187-191 (1985) reviews new methodologies for fusing cells in electric fields and states that Lundak and his associates at Techniclone isolated the **myc** gene from human malignant cells, cloned it, inserted it into a plasmid and used a high voltage electric pulse to transfect antibody - producing human lymphocytes with the plasmid to produce a human cell line that has been stable for at least eight months. The Van Brunt reference does not enable one of skill in the art to repeat Lundak's experiment because there is no disclosure regarding (a) what voltage pulse must be employed, (b) whether or not such cells must be "activated/stimulated" prior to transfection, (c) how to being the cells and plasmid in close proximity prior to pulsing, (d) how many pulses to give, (e) the appropriate length and amplitude of each pulse, (f) the appropriate concentrations of cells and oncogenic DNA, (g) the nature and origin of the "myc" gene which was employed, and (h) tissue culture conditions under which such cells will proliferate in culture, and maintain its proliferation indefinitely.

## SUMMARY OF THE INVENTION

This invention relates to a method for production of an immortalized primary cell line which comprises:

(a) providing normal, activated/stimulated primary cells;

(b) transfecting such primary cells with oncogenic DNA/oncogene(s) by electroporation thereby producing transfected primary cells; and

(c) recovering from such transfected primary cells an immortalized primary cell line.

The oncogenic DNA/oncogenes used to transfect the primary cells in step (b), above, may be optionally comprised by liposomes prior to the transfection by electroporation.

This invention also relates to the immortalized primary cell line produced by this method.

This invention also relates to a method of producing biological products whose coding sequence is comprised by the DNA contained within the immortalized primary cell line of this invention which comprises culturing such cell line under conditions which enable the production of such biological products. The term "coding sequence" includes those coding sequences which are

naturally found within the DNA of the normal, activated/stimulated primary cell used to prepare the immortalized primary cell line of this invention as well as the coding sequence(s) comprised by the oncogenic DNA/oncogene(s) used in the method of this invention.

This invention also relates to a method for production of a cotransfected immortalized primary cell line containing a foreign functional DNA sequence which comprises:

(a) providing normal, activated/stimulated primary cells;

(b) cotransfecting such primary cells with oncogenic DNA/oncogene(s) and the foreign functional DNA sequence by electroporation thereby producing cotransfected primary cells; and

(c) recovering from such cotransfected primary cells a cotransfected immortalized primary cell line containing the foreign functional DNA sequence.

This invention also relates to the cotransfected immortalized primary cell line produced by this method.

This invention also relates to a method of producing biological products whose "coding sequence" is comprised by the DNA contained within the cotransfected immortalized primary cell line of this invention which comprises culturing such cell line under conditions which enable the production of such biological products.

The term "coding sequence" includes the coding sequence(s) of the foreign functional DNA sequence(s) comprised by the oncogenic DNA/oncogene(s) used for transfection in the method of this invention, as well as those DNA coding sequences which are "naturally found" within the DNA of the normal, activated/stimulated primary cell used as DNA recipient cells to prepare the contransfected immortalized primary cell line(s) of this invention.

This invention also relates to an immortalized primary cell line provided that such immortalization is the result of transfection of a normal primary cell with DNA consisting essentially of oncogene(s).

The oncogenic DNA and foreign functional DNA used to transfect the primary cells in step (b), above, may be optionally comprised by liposomes prior to the transfection by electroporation.

## DETAILED DESCRIPTION OF THE INVENTION

Jonak et al., Hybridoma, 3, 107-118 (1984) report that the introduction of DNA from human leukemia cells into stimulated mouse lymphocytes by calcium phosphate DNA-coprecipitation/polyethylene glycol-DMSO mediated delivery can produce cell lines with the capacity to proliferate continuously in culture. It has now been discovered that transfection of primary cells such as immune cells, including lymphocytes, with oncogenic DNA to effect immortalization of such cells depends on the methodology used to deliver such DNA into the cells, the functional state of the recipient cell and the source of oncogenic DNA used to bestow immortalization. For example, the calcium phosphate DNA coprecipitation technique in which B lymphocytes are incubated with the coprecipitate and the DNA entry is enhanced by polyethylene glycol-DMSO shock treatment (Jonak et al., Hybridoma, cited above) was employed for transfection of oncogenic DNA into a variety of primary immune cells (i.e., cells of hematopoietic origin) to attempt to immortalize such cells. This approach was only randomly successful in its efficiency of producing immortalized cell lines. Furthermore, the calcium phosphate-DNA coprecipitation technique has additional limitations which are related to the nature of the recipient cells. For example, primary cells (with the exception of macrophages and certain cells of fibroblastic origin with endocytotic potential) are not phagocytic, therefore the uptake of calcium phosphate-DNA coprecipitate is not a very efficient method of DNA introduction into these cells. Also, another limitation of the calcium phosphate-DNA coprecipitation method is that it is elaborate and involves the exposure of primary immune cells to a high concentration of salts which has a direct effect on the viability of these cells and interferes with the transfection efficiency of such method.

It has now been discovered that electroporation can be reliably employed to efficiently transfect normal, activated/stimulated primary cells of both hematopoietic and non-hematopoietic origin with oncogenic DNA/oncogenes to efficiently create immortalized primary cell lines. It has also now been discovered that electroporation can be reliably employed to efficiently transfect normal, activated/stimulated primary cells of both hematopoietic and non-hematopoietic origin with oncogene(s) to efficiently create immortalized primary cell lines.

By the term "primary cell" is meant any cell obtained from a living organism which was not previously passed in tissue culture or has been previously passed in tissue culture but is incapable of being indefinitely passed in tissue culture. Preferred primary cells are those of hematopoietic origin, particularly multipotential stem cells, B-lymphocytes, B-lineage precursors, T-lymphocytes, T-lineage precursors, myeloid stem cells, monocytes, macrophages, neutrophils, eosinophils, megakaryocytes, and mast cells. Such cells can be isolated by conventional techniques.

By the term "immortalized primary cell" is meant a primary cell which proliferates continuously in culture as a result of transfection by the method of this invention, and which without such transfection would not so proliferate.

By the term "normal primary cell" is meant a non-immortalized primary cell that can be made capable of immortalization by the method of this invention if such normal primary cell is first activated/stimulated. By the term "activated/stimulated primary cell" is meant a normal primary cell which has been made capable of immortalization by the method of this invention. Such activated/stimulated primary cell can be prepared by exposure of such cell to a stimulating/activating agent. By the term "stimulating/activating agent" is meant an agent which will stimulate the normal primary cell to proliferate (limited life span). Such agents are well known in the art. Preparation of an activated/stimulated primary cell is well known in the art. Preferably, the stimulating/ activating agent will also stimulate the normal primary cell to differentiate. Appropriate stimulating/activating agents to be employed to activate/stimulate normal B cells include antigens, mitogens and growth factors. If production of a specific monoclonal antibody by the immortalized primary cell line of this invention is desired, then a known antigen should be employed as the stimulating/activating agent. For example, an activated/ stimulated B lymphocyte is a lymphocyte that has been challenged by an antigen and has differentiated into an antibody-producing cell, the antibody being specific for one antigenic determinant of the challenging antigen. Such activated/stimulated B lymphocyte is now capable of immortalization by the method of this invention. Appropriate stimulating/activating agents to be employed to activate/stimulate normal primary cells other than B cells include mitogens and growth factors. Mitogens are well known in the art. It should be noted that specific mitogens may be preferred for particular types of normal primary cells. For example, lipopolysaccharide (LPS) is a preferred mitogen for stimulating/activating a normal B cell; Concanavalin A is a preferred mitogen for stimulating/activating a normal T cell (it stimulates differentiation as well as proliferation); pokeweed mitogen is a preferred mitogen for stimulating/activating either a normal B cell or a normal T cell (it stimulates differentiation as well as proliferation); insulin and polymyristic acid (PMA) are preferred mitogens for stimulating/activating a normal, fibroblast cell.

The optimal stimulating/activating agent to employ and the optimal conditions for stimulation/activation of a normal primary cell can be determined by one of skill in the art employing conventional techniques, and will depend upon, among other factors, the primary cell type employed and the particular stimulating/activating agent.

By the term oncogenic DNA/oncogene(s) is meant any DNA sequence(s) which will immortalize a normal, activated primary cell when transfected into such cell by the method of this invention. "Oncogenic DNA" as used herein includes that DNA from tumor cells or tumor cell lines which will immortalize a normal, activated/stimulated primary cell when transfected into such cell by the method of this invention. An oncogene is an isolated DNA sequence known for its potential to play a role in transforming a normal cell into a cancer cell. "Oncogenes" as used herein includes any isolated DNA sequence(s) which will immortalize a normal, activated/stimulated primary cell when transfected into such cell by the method of this invention. It is important to note that more than one oncogene type may be required to immortalize a particular normal, activated primary cell according to the method of this invention. Preferred oncogene(s) to be employed to immortalize a normal, activated, stimulated primary cell according to the method of this invention includes oncogene(s) from a primary cell tumor or a tumor primary-cell line wherein such tumor or tumor cell line has an origin corresponding to the primary cell type to be immortalized. The preferred oncogene to be employed to immortalize a normal, activated/stimulated B cell according to the method of this invention is the Ha-ras oncogene isolated from the T24 human bladder carcinoma cell line available from the American Type Culture Collection, Rockville, Maryland, U.S.A. (ATCC) under accession number ATCC HTB4 ("Ha-ras") and p53 oncogene (clone p53-H7 this clone was obtained from Dr. Varda Rotton; detailed description of this clone is in Molecular and Cellular Biology, Vol.5, No. 8, pages 1887-1893, 1985). Preferred oncogenic DNA to be employed to immortalize a normal, activated/stimulated B cell according to the method of this invention includes genomic DNA from the acute human lymphoblastic leukemia known as the REH cell line available from the ATCC under accession number CRL8286 or the EL4 murine thymoma cell line (EL4) available from the ATCC under accession number ATCC TIB39; and genomic DNA from a tumor B-cell line or a B cell tumor, particularly B cell lymphoma such as the human Burkitt's lymphoma cell line (BJAB) of Dr. W. Henle, Children's Hospital, Department of Virology, Philadelphia, PA, U.S.A. Preferred oncogenic DNA to be employed to immortalize a normal, activated/stimulated T cell according to the method of this invention includes genomic DNA from a T cell tumor or a tumor T-cell line such as EL4. Preferred oncogenic DNA to be employed to immortalize other normal, activated/stimulated primary cells according to the method of this invention includes genomic DNA from a primary cell tumor or a tumor primary-cell line wherein such tumor or tumor cell line has an origin corresponding to the normal primary cell type to be immortalized. For example, tumor DNA of epithelial origin is preferred for immortalizing normal, activated-stimulated primary epithelial cells according to the method of this invention. Other appropriate oncogenic DNA/oncogene(s) to be employed to prepare the immortalized cell line of this invention can be determined by one of skill using the method of this invention, and will depend upon many factors such as the source of the oncogenic DNA/oncogene(s) and the normal, activated primary cell type to be employed. Many different oncogenic DNA/oncogene(s) have been reported recently in the literature, and some oncogenic DNA/oncogene(s) are publicly available from various sources such as the ATCC.

By the term "electroporation" is meant exposure of a cell to a high-voltage electric pulse. Such exposure renders the cell membrane more permeable to certain high molecular weight macromolecules such as DNA. Electroporation can be effected by any apparatus capable of emitting a high-voltage electric pulse to a container comprising cells and DNA. For example, there are many commercially available electro-fusion systems such as the BTX Electro Cell Manipulator, Model 401 available (from BTX, San Diego, California, U.S.A.) which are readily adaptable to an electroporation apparatus by one of skill in the art. Furthermore, electroporation may be effected by the method of Potter et al., Proc. Natl, Acad. Sci., USA, 81 7161-6175 (1984). If the Potter method is employed, LKB power supply#2197 (LKB Company, Sweden) is the preferred source of the electric pulse.

By the term "high-voltage" is meant an electric voltage of between about 1,000 volts/cm to about 5,500 volts/cm. The optimal voltage can be determined by one of skill in the art and will depend upon various factors such as the nature of the normal, activated/stimulated primary cells, nature of oncogenic DNA/oncogene(s) source, cell and/or DNA concentration cell density and the nature of the electroporation apparatus employed.

This invention relates to a method for production of an immortalized primary cell line which comprises:

(a) providing normal, activated/stimulated primary cells;

(b) transfecting such primary cells with oncogenic DNA/oncogene(s) by electroporation thereby producing transfected primary cells; and

(c) recovering from such transfected primary cells as immortalized primary cell line.

This invention also relates to the immortalized primary cell line produced by the method of this invention.

The general methodology employed to transfect oncogenic DNA/oncogene(s) into normal, activated/stimulated primary cells by electroporation is as follows. Normal primary cells are isolated by conventional techniques. They are activated/stimulated after isolation as outlined above (in vivo stimulation), or can be isolated as normal, activated/stimulated primary cells from a donor (living organism) which was exposed $\underline{in}$ $\underline{vivo}$ to an appropriate stimulating/activating agent. Such normal, activated/stimulated cells are then suspended in a protective medium, preferably from about $5x10^6$ to about $2x10^7$ cells/ml.

By the term "protective" medium is meant any medium which maintains the viability of the normal, activated/stimulated primary cells during electroporation. Examples of protective media which may be employed in the method of the subject invention include buffered solutions and non-ionic solutions such as phosphate-buffered saline (PBS) (pH ca. 7) and glucose (.3 molar). PBS is most preferred. If PBS is employed, the preferred voltage is 1000 volts/cm. If glucose (.3 molar) is employed, the preferred voltage is 5500 volts/cm.

After suspension of the normal, activated/ stimulated primary cells in the protective medium, oncogenic DNA (preferably from about 1 ug/ml to about 100 ug/ml) or oncogene(s) (10 ng/ml to about 10 ug/ml) is added to the suspension. The oncogenic DNA/oncogene(s) added to the suspension may be in the form of free DNA, comprised of genomic DNA or an appropriate recombinant DNA vector, or DNA can be encapsulated within a liposome which is coated with antibodies specific for at least one antigenic determinant on the normal, activated/stimulated primary cell surface. By "appropriate recombinant DNA vector" is meant a vector prepared by recombinant DNA techniques which contains an expression control sequence operatively linked to the oncogenic DNA/oncogene(s) it comprises. Preparation of recombinant DNA vectors is well known in the art. By "expression control sequence" is meant a DNA sequence, such as a promoter which regulates transcription of the oncogenic DNA/oncogene(s). By the term "promoter" is meant any region upstream of the oncogenic DNA/oncogene(s) which permits binding of RNA polymerase and transcription to occur. By "operatively liked" is meant that the oncogenic DNA/oncogene(s) is transcriptionally or translationally linked to an appropriate expression control sequence. Preparation of liposomes and preparation of antibody coated liposomes is well known in the art.

Preferably, the suspension containing both the normal, activated/stimulated primary cells and the oncogenic DNA/oncogene(s) is allowed to incubate for about 5 to about 10 minutes at about 2 to 4°C prior to exposing the suspension to electroporation. Then, one to ten, preferably three to five, high voltage electric pulses are delivered to the suspension, thereby transfecting the normal, activated/stimulated primary cells with the oncogenic DNA/oncogene(s). The preferred length of the electric pulse on the BTX Electro Cell Manipulator, Model 401 is from about 50 to about 99 microseconds, 99 microseconds being most preferred. The preferred amplitude of the electric pulse on the BTX Electro Cell Manipulator, Model 401 is the maximum which it can be. The optimal voltage, length, amplitude and number of electric pulses to deliver to the cells/DNA suspension can be determined by one of skill in the art by employing the method of this invention and will depend upon various factors, such as the nature of the primary cells employed, the nature of the oncogenic DNA/oncogene(s) source, the concentration of the primary cells in the suspension, the concentration of the oncogenic DNA/oncogene(s) in the suspension the protective medium employed and the nature of the electroporation apparatus employed.

Preferably, after electroporation has been effected, the suspension is allowed to sit for about 5 to about 10 minutes at about 2 to about 4°C before fresh medium is added to nourish the transfected primary cells.

After electroporation has been effected, the immortalized primary cell line of this invention is recovered from the suspension containing the transfected primary cells by employing conventional techniques for identifying immortalized cell lines. For example, the cell suspension is distributed to 96 well plates and fed with fresh medium once a week or as needed. Those wells which contain clones of cells, showing continuous proliferation (designated as number of cell clones), for example, proliferation for at least 6 weeks, contain the immortalized, primary cell lines of this invention.

After recovery, the immortalized primary cell line of this invention may be clonally expanded by conventional techniques to produce a homogenous population of such cell lines which can be maintained, continuously, in the appropriate culture medium, i.e., a medium which will maintain the viability of such cell lines and enable them to proliferate.

This invention also relates to a method of producing biological products whose coding sequence is comprised by the DNA contained within the immortalized primary cell line of this invention which comprises culturing such cell line under conditions which enable the production of such biological products. As stated above, the term "coding sequence" includes those coding sequences which are naturally found within the DNA of the normal, activated/stimulted primary cell used to prepare the immortalized primary cell line of this invention as well as the coding sequence(s) comprised by the oncogenic DNA/oncogene(s) used in the method of this invention. All normal primary cells are potentially capable of producing biological products whose coding sequence is comprised by the DNA contained within such primary cell. Activation/stimulation of such primary cells may enable them to produce different or additional biological products. For example, after activation/stimulation of a normal B lymphocyte with an antigen of interest, such normal, activated/stimulated B cell can differentiate into an antibody producing cell. The antibody produced will be specific for one determinant of the activating/stimulating antigen. Immortalization of such normal, activated/stimulated B lymphocyte by the method of this invention will produce a cell line capable of providing a continuous source for monoclonal antibody production, as well as the product(s) of the oncogenic DNA/oncogene coding sequence(s) comprised by such immortalized primary cell line. Likewise, immortalization of other normal primary cells enables them to continuously produce (a) a biological product whose coding sequence is comprised by the DNA contained within such primary cells; for example, such coding sequence may code for a lymphokine, for example, interleukin-1 (IL-1), interleukin-2 (IL-2), or interferon (IF); or for a growth factor, as well as (b) the product(s) of the oncogenic DNA/oncogene coding sequence(s) comprised by such immortalized primary cell lines. The appropriate medium conditions under which to culture the immortalized primary cell line of this invention to enable it to produce a desired biological product (it is capable of producing) can be readily determined by one of skill in the art by conventional techniques and will depend on, among other things, the type of primary cell line involved and the desired biological product to be produced. The biological product(s) produced by the primary cell lines of this invention according to the method of this invention might be secreted by such cell line directly into the culture medium, in which case such biological product(s) may be isolated from such culture medium, if isolation is desired, by one of skill in the art employing conventional protein isolation techniques. The biological product(s) also might not be secreted, in which case such biological product(s) would be obtained, if isolation is desired, from a culture lysate of such cell lines by employing conventional techniques.

This invention also relates to a method for production of a cotransfected immortalized primary cell line containing a foreign functional DNA sequence which comprises:

(a) providing normal, activated/stimulated primary cells;

(b) cotransfecting such primary cells with oncogenic DNA/oncogene(s) and the foreign functional DNA sequence by electroporation thereby producing cotransfected primary cells; and

(c) recovering from such cotransfected primary cells an immortalized primary cell line containing the foreign functional DNA sequence.

This invention also relates to the cotransfected, immortalized primary cell line produced by the method of this invention.

By the term "functional DNA sequence" is meant any discrete region of DNA derived from any source which functions in the cotransfected immortalized primary cell line of this invention as a gene expression unit, structural gene, promotor or a regulatory region. By the term "gene expression unit" is meant a structural gene and the promotor and regulatory regions required for its transcription and translation. By the term "promotor" is meant any region upstream of a structural gene which permits binding of RNA polymerase and transcription to occur. By the term "regulatory region" is meant a DNA sequence which regulates transcription of a structural gene. By the term "structural gene "is meant a coding sequence for a polypeptide which serves to be the template for the synthesis of MRNA.

By the term "foreign functional DNA sequence" is meant a functional DNA sequence which is not naturally present in the DNA of the normal, activated/stimulated primary cell used in the method of this invention and which is not part of the oncogenic DNA/oncogene(s) responsible for immortalization of such cell according to the method of this invention. Preferred foreign functional DNA sequences include those coding for any polypeptide product of pharmaceutical importance such as, but not limited to, insulin, growth hormone, tissue plasminogen activator, -1-antitrypsin, hirudin, antigens used in vaccine production, lymphokines, growth factors, and receptors, as well as DNA sequences coding for products capable of enhancing the proliferation of the cotransfected immortalized primary cell of this invention.

The general methodology employed to produce the cotransfected immortalized primary cell line of this invention is substantially the same as that employed to produce the immortalized primary cell line of this invention with the exception that it involves the cotransfection of oncogenic DNA/oncogene(s) and a foreign functional DNA sequence. Such cotransfection may be carried out simultaneously or sequentially. By "sequentially" is meant that the activated/stimulated primary cell can be first made into an immortalized primary cell line by the method of this invention and then later transfected with the foreign functional DNA sequence by electroporation or any other DNA transfection technique. The preferred amount of foreign functional DNA to employ is from about 1 ug/ml to about 10oug/ml. The optimal amount of the foreign functional DNA to employ can be determined by one of skill in the art using the method of this invention and will depend on the nature of the foreign functional DNA employed and the other factors outlined above with regard to the method for producing the immortalized primary cells of this invention. Such foreign functional DNA sequence may be added to the suspension as free DNA, incorporated into an appropriate recombinant DNA vector (which may also contain the oncogenic DNA/oncogene(s) to be employed), or encompassed within a liposome bearing antibodies to antigenic determinants on the surface of the primary cell to be employed in the cotransfection. The preferred conditions of electroporation to utilize for such cotransfection, i.e., the number amplitude, voltage and length of the electric pulses, are the same as those outlined about regarding preparation of the immortalized primary cell line of this invention.

After electroporation has been effected, the cotransfected immortalized primary cell line of this invention is recovered from the suspension by (a) employing conventional techniques for identifying immortalized cell lines such as those outlined above, and (b) by employing conventional techniques to identify which of such immortalized cell lines comprise the foreign functional DNA sequence.

After recovery, the cotransfected immortalized primary cell line of this invention may be clonally expanded by conventional techniques to produce a homogeneous population of such cell lines which can be maintained, continuously, in the appropriate culture medium, i.e., a medium which will enable such cell lines to remain viable and continue to proliferate. Such culture medium can be determined by one of skill in the art.

This invention also relates to a method of producing biological products whose coding sequence is comprised by the DNA contained within the cotransfected immortalized primary cell line of this invention which comprises growing such cell line under conditions which enable the production of such biological products. As stated above, the term "coding sequence" includes the coding sequence(s) of the foreign functional DNA sequence as well as those coding sequences which are naturally found within the DNA of the normal, activated/stimulated primary cell used to prepare the cotransfected immortalized primary cell line of this invention as well as the coding sequence(s) comprised by the oncogenic DNA/oncogene(s) used in such cell line's preparation.

The appropriate medium conditions under which to culture the cotransfected immortalized primary cell of this invention to enable it to produce a desired biological product (it is capable of producing) can be readily determined by one of skill in the art by conventional techniques and will depend on, among other things, the type of primary cell line involved and the

- 21 -

0235113

desired biological product to be produced. The biological products produced by the cotransfected immortalized primary cell line of this invention according to the method of this invention may be either secreted directly into the culture medium by such cell line, in which case such biological products may be isolated from such culture medium, if isolation is desired, by one of skill in the art employing conventional protein isolation techniques. The biological products may not be secreted by such cell line, in which case such biological products would be obtained, if isolation is desired, from a culture lysate of such cell lines by employing conventional techniques.

Therefore, the cotransfected immortalized primary cell line of this invention provides a continuous source for (a) the polypeptides coded for by the DNA naturally found in the normal, activated/stimulated primary cell used in the method of this invention to prepare the cell line; (b) the polypeptide(s) coded for by oncogenic DNA/oncogene(s) used in the method of this invention, and (c) the polypeptide(s) coded for by the foreign functional DNA sequence comprised by the cotransfected immortalized primary cell line of this invention.

It has also now been discovered that DNA consisting essentially of oncogene(s) can be employed to immortalize a normal, primary cell. Thus, this invention also relates to an immortalized primary cell line provided that such immortalization is the result of transfection of a normal primary cell with DNA consisting essentially of oncogene(s). The preferred method of preparing such immortalized primary cell line is by electroporation-mediated transfection of a normal, primary cell according to the method of this invention.

## EXAMPLES

In the following Examples, specific embodiments of the invention are more fully disclosed. These Examples

- 22 -                        0235113

are intended to be illustrative of the subject invention and should not be construed as limiting its scope.

## EXAMPLE 1
### Stimulation/activation of lymphocytes

Lymphocyte stimulation/activation is achieved by conventional techniques such as by employing the following general methodology:

(a) Normal human or mouse lymphocytes are activated/stimulated in vivo with the desired antigen (quantity varied) by injecting such antigen into animals by various routes. The period between activation/stimulation and transfection, by the method of this invention, as well as the number of injections may be varied. The normal, stimulated/activated lymphocytes are removed from the mouse spleen and treated as reported by Kennett et al., Current Topics in Microbiol. and Immunol., 81, 77-94 (1978).

(b) Normal human or mouse lymphocytes are stimulated/activated in vitro by using antigen or mitogen as the stimulating/activating agent. The in vitro procedure for stimulation/activation with the specific antigen is the modified method as described by Jonak et al., Hybridoma, 3, 107-118 (1984).

(c) Normal Human or mouse lymphocytes, primed in vivo are restimulated by in vitro with desired antigen/mitogen.

## EXAMPLE 2
### Immortalization of human lymphocytes

As a general exemplification of the production of immortalized primary cells with oncogenic DNA/oncogene(s) by the method of this invention, a suspension of normal human lymphocytes and oncogenic DNA is mixed and exposed to a high-voltage electric pulse according to the following protocol:

Normal, human lymphocytes (peripheral blood lymphocytes) are purified, using lymphoprep, by centrifugation. Cells are activated/stimulated as described in Example 1. Such normal activated/stimulated primary cells are suspended in ice-cold phosphate-buffered saline (PBS, without added $MgCl_2$ or $CaCl_2$) and centrifuged again (200 x g/10 minutes). The pellet is resuspended in PBS at a concentration of $1-5x10^7$ cells per ml. Genomic DNA (40-50 ug/ml) purified from a human tumor cell line (Burkitt's lymphoma, BJAB) whose origin corresponds to the B-lymphocyte cell type or plasmid DNA (p53-H7) containing functional oncogene(s) (1-20 ug/ml) is added to the cell suspension and allowed to incubate for 10 minutes at 0°C in the electroporation chamber of a BTX Electro Cell Manipulator, model 401. Three to five electric pulses (1,000 volts/cm to 5,500 volts/cm) (maximum amplitude) (99 microseconds in length) are delivered by the BTX Electro Cell Manipulator, model 401.

After the delivery of the electric pulses, the cells and DNA are allowed to sit for 10 minutes at 0°C before being added to 5 ml of conditioned TY medium (Jonak et al., "Monoclonal Antibodies and Functional Cell Lines, "Plerum Press, 418-422, 1984) and distributed to 96 well plates. The cells are fed with fresh medium once a week or as needed.

Successful high efficiency immortalization was achieved in experiments in which human lymphocytes were transfected by electroporation with oncogenic DNA from BJAB human Burkitt's lymphoma cell line or with oncogene (functional oncogene(s) inserted into expression vectors) p53. This success was clearly evidenced by the high number of continuously proliferating cells which were obtained. In control experiments, the lympho-

cycles were treated identically except that the oncogenic DNA/oncogene(s) was substituted by normal DNA or was omitted. Comparisons of the cultures transfected with oncogenic DNA/oncogene(s) to the controls indicated that the number of cells showing proliferation (designated as number of clones) was much higher with oncogenic DNA/oncogene(s). Cells in the control experiments died within three weeks. Oncogenic DNA/oncogene transfected cells showed continuous proliferation for over six weeks to several months thereby confirming their immortalization.

### EXAMPLE 3

## Immortalization of mouse lymphocytes

Spleen cells from Balb/c mice (female from Charles River Laboratories) were prepared by perfusion of whole spleen and treated with 0.83% $NH_4Cl$ at $4^{\circ}C$ for 5 minutes in order to lyse red blood cells. Lymphocytes were then centrifuged and the pellet resuspended with 5 ml of Dulbecco's modified EAGLE's medium with high glucose (DMEM) (see, Cerottini et al., J. Exp. Med., 140, 703-717 (1974) and applied on a nylon wool column (packed nylon wool on the column was 10 ml). The column was kept at $37^{\circ}C$ for 45 minutes and T lymphocytes were removed by filtration through this column by adding 20 ml of DMEM medium supplemented with 5% fetal calf serum (FCS) warmed to $37^{\circ}C$ (Julius et al., Eur. J. Immunol., 3:645, 1973). B lymphocytes were then recovered from the column by mixing the nylon wool with 20 ml of the same medium. Cells were centrifuged and the pellet (about $4x10^7$ B cells from one spleen) was resuspended with Iscove's modification of DMEM (RPMI 1640) (See, Moore et al., GAMA, 199, 519-524 (1967) supplemented with 5% FCS. Cells (3 x $10^7$) were incubated at $37^{\circ}C$ in 7% $CO_2$ in tissue culture flasks with 20 ml of RPMI 1640 medium supplemented with 5% heat inactivated FCS, 2 mM glutamine, $5x10^{-5}$ M 2-mercaptoethanol and gentamycin.

For mitogen-induced stimulation, purified B lymphocytes were incubated with 20 ug/ml of lipopolysaccharide (LPS). The maximum stimulation was achieved after cells were stimulated for 24-48 hours with LPS ($^3$(H)-thymidine incorporation into the DNA was used to indicate the degree of stimulation). After 24-48 hours of culture, cells (lymphoblasts) were washed two times with PBS buffer (without $CaCl_2$ and $MgCl_2$) and resuspended at a cell concentration of $2x10^6$-$2x10^7$ cells/ml and kept at $0^{\circ}$C. The source of oncogenic DNA was purified genomic DNA from EL4 murine thymoma cell line (ATCC TIB39) or oncogene Ha-ras [isolated from T24 (ATCC HTB4)] inserted in a proper expression vector. The electroporation was achieved by (a) suspending $10^6$-$10^7$ cells/0.5 ml of PBS with 40 ug of genomic DNA or 10-100 ng of oncogene in the electroporation chamber of a BTX Cell Manipulator, Model 401, and (b) delivering 3 to 5 electric pulses (maximum length and amplitude). After electroporation, the cells were left at $0^{\circ}$C for 10 minutes before diluting them with TY medium [See, Jonak et al., "Monoclonal Antibodies and Functional Cell Lines (Progress and Applications), "Plenum Press, 418-422 (1984)] at a concentration of $10^6$ cells/ml. Cells were then incubated in 96 well plates at a concentration of $2x10^5$ cells/well and incubated at $37^{\circ}$C. Each week cells were fed with fresh medium. After 3 to 4 weeks of culture, wells showing growing cells were expanded. Such cells had blast morphology and grew for several months in culture. The results of these experiments indicated that the stimulation played an essential role in the immortalization since unstimulated lymphocytes did not grow after electroporation-mediated transfection with oncogenic DNA/oncogene(s).

## EXAMPLE 4

### Electroporation/DNA-liposome delivery

The electroporation technique of the subject invention can combine the use of antibody/protein A-bearing liposomes containing oncogene(s)/oncogenic DNA with the electroporation.

Large unilamellar vesicles of lipid composition of 54 mol% dimyristoylphosphatidylcholine (Avanti polar lipid), 10 mol% dimyristoylphosphatidylserine (Avanti), 35 mol% cholesterol (Sigma and 1 mol% dipalmitoylphophatidylethanolamie (Sigma) modified with N-hydroxysuccinimidyl 3-(2-pyridyldithio propionate) (SPDP) (Pharmacia) were made according to a modification of the reverse phase evaporation technique (Szoka and Papahadjopoulos, Proc. Natl. Acad. Sci., USA, 75:4194-4198, (1978). Lipids were dissolved in 1 ml chloroform/2 ml diisoproyl ether (Merck). The aqueous (1 ml containing 1 mg Fo2mg of plasmid DNA with a radiolabeled aliquot in buffer saline) and organic phases were placed in separate gas-tight glass syringes fitted with luer locks as already described (Machy and Leserman, Biophys. Biochim. Acta, 730:313-320, (1983), Machy and Leserman, "Liposome Technology" (Gregoriadis, ed) CRC Press, Cleveland, Vol. I:221-234, (1984). The syringes were joined by a metal three-way stopcock. The squeous phase was injected into organic phase, then the mixture was passed rapidly 10-20 times from syringe to syringe. The lipid-aqueous emulsion was transferred to a 14 ml rotovap tube and warmed to 45°C, and the organic phase was removed at 350-400 mm Hg pressure. Reverse-phase evaporation vesicles (REV) were then selected on the basis of size by filtration through 0.4 um. Unipore polycarbonate filters (Bio-Rad) (Olson et al., Biochim. Biophys. Acta, 557:9-23 (1979).

Liposomes were incubated with protein A (Pharmacia) with an eliquot of iodinated protein A (NEN), modified by 10 mol SPDP/mol protein A (final concentration 200 ug/ml) (Leserman et al., Nature, 288:602-604, (1980); Machy et al., J. Immunol., 129:2098-2102, (1982). The final concentration of lipid was around 20mM. The coupling reaction was performed in L-buffer (145 ml NACl, 10 mM Hepes, pH 8) for 20 hours at room temperature. Prior to the binding the DNA-containing protein A-coated liposomes were purified as described: Liposomes were incubated for 30 minutes at 25°C with 50 ug/ml of DNase I (Sigma) in L-buffer supplemented with 10 ml MgClMgCl$_2$. Protein A coated-liposomes that contained DNA were then passed through a Sepharose 4B-CL equilibrated with L-buffer (pH 7.45). Uncoupled protein A and degraded unencapsulated DNA were thus eliminated from the liposome preparation. Usually about 5% of DNA was encapsulated and 5-10% of protein A coupled to the surface of liposomes.

Such preferred liposomes are incubated with lymphoid as described:

Human B-cells ($10^7$) are incubated at 4°C for 1 hour with 40 ug/ml of monoclonal antibody. The cells are then washed to remove the unbound antibody and incubated for 2 hours at 4°C with 3 ug of DNA containing protein A-coated liposomes. Liposomes bind to the antibody pretreated cells. The cells are then electroporated as described before. As an example at least 35% of the wells showed transfected cells when these cells were incubated with an anti-HCA antibody prior to addition of liposomes. With an irrelevant antibody or without antibody only 8-10% of the wells showed transfected cells. When cells were incubated with the anti-HLA antibody and liposomes but not electroporated, no cells were transfected. The conclusion of this example is that only targeted liposomes can transfect the cells by using electroporation.

The advantage of this approach is that the DNA (encapsulated into liposomes) will be specifically delivered to the desired cell type (e.g. mature B-cell, monocyte) in the mixed population of cells. The content of the liposomes [oncogenic DNA/oncogene(s)] will be introduced to the cell by applying an electric pulse. It should be pointed out that it is not expected or desired that the liposomes fuse with the target cells, but rather that by applying the electric pulse, the DNA is inserted into the cell through the pores (which result from the electric pulse) formed between the target cell and the liposome. The advantage of this modification is the quantity of DNA delivered specifically to the desired cell types in a mixed population of lymphocytes.

While the above descriptions and Examples fully describe the invention and the preferred embodiments thereof, it is understood that the invention is not limited to the particular disclosed embodiments. Thus, the invention includes all embodiments coming within the scope of the following claims.

Claims for the Contracting States :
BE, CH, DE, FR, GB, IT, LI, LU, NL, SE


1.   A method for producing an immortalized primary cell line which comprises:

(a) providing normal, activated/stimulated primary cells;

(b) transfecting such primary cells with oncogenic DNA/oncogene(s) (which, optionally, is comprised by liposomes) by electroporation thereby producing transfected primary cells; and

(c) recovering from such transfected primary cells an immortalized primary cell line.

2.   The method of Claim 1 wherein the oncogenic DNA is genomic DNA from a primary cell tumor or a tumor primary-cell line wherein such tumor or tumor cell-line has an origin corresponding to the primary cell type to be immortalized.

3.   The method of Claim 1 wherein the oncogene(s) is/are from a primary cell tumor or a tumor primary cell line wherein such tumor or tumor cell line has an origin corresponding to the primary cell type to be immortalized.

4.   The method of Claim 2 wherein the primary cells are of hematopoietic origin.

5.   The method of Claim 3 wherein the primary cells are multipotential stem cells, B-lymphocytes, B-lineage precursors, T-lympocytes, T-lineage precursors, myeloid stem cells, monocytes, macrophages, neutrophils, eosinophils, megakaryocytes or mast cells.

6. The method of Claim 4 wherein the primary cells are B-lymphocytes.

7. The method of Claim 5 wherein the B lymphocytes have been activated/stimulated with an antigen.

8. The method of Claim 5 wherein the oncogene(s) is the Ha-ras oncogene or the p53-H7 oncogene.

9. The method of Claim 6 wherein the oncogenic DNA is genomic DNA from BJAB.

10. The method of Claim 6 wherein the oncogenic DNA is genomic DNA from the REH cell line.

11. The method of Claim 6 wherein the oncogenic DNA is genomic DNA from the EL4 cell line.

12. An immortalized primary cell line produced by the method of Claim 1.

13. The cell line of Claim 12 wherein the oncogenic DNA is genomic DNA from a primary cell tumor or a tumor primary-cell line wherein such tumor or tumor cell-line has an origin corresponding to the primary cell type to be immortalized.

14. The cell line of Claim 12 wherein the oncogene(s) is/are from a primary cell tumor or a tumor primary cell line wherein such tumor or tumor cell line has an origin corresponding to the primary cell type to be immortalized.

15.    The cell line of Claim 12 wherein the primary cells are of hematopoietic origin.

16.    The cell line of Claim 15 wherein the primary cells are multipotential stem cells, B-lymphocytes, B-lineage precursors, T-lympocytes, T-lineage precursors, myeloid stem cells, monocytes, macrophages, neutrophils, eosinophils, megakaryocytes or mast cells.

17.    The cell line of Claim 16 wherein the primary cells are B-lymphocytes.

18.    The cell line of Claim 17 wherein the B lymphocytes have been activated/stimulated with an antigen.

19.    The cell line of Claim 16 wherein the oncogene(s) is the Ha-ras oncogene.

20.    The cell line of Claim 16 wherein oncogenic DNA is genomic DNA from BJAB.

21.    The cell line of Claim 16 wherein the oncogenic DNA is genomic DNA from the REH cell line.

22.    The cell line of Claim 16 wherein the oncogenic DNA is genomic DNA from the EL4 cell line.

23.    A method of producing biological products whose coding sequence is comprised by the DNA contained within an immortalized primary cell line which comprises:

(a) providing normal, activated/stimulated primary cells;

(b) transfecting such primary cells with oncogenic DNA/oncogene(s) (which, optionally is comprised by liposomes) by electroporation thereby producing transfected primary cells;

(c) recovering from such transfected primary cells the immortalized primary cell line; and

(d) culturing such immortalized cell line under conditions which enable the production of such biological products.

24. A method for production of a contransfected immortalized primary cell line containing a foreign functional DNA sequence which comprises:

(a) providing normal, activated/stimulated primary cells;

(b) cotransfecting such primary cells with oncogenic DNA/oncogene(s) and the foreign functional DNA sequence (which, optionally, are comprised by liposomes) by electroporation thereby producing cotransfected primary cells; and

(c) recovering from such cotransfected primary cells an immortalized primary cell line containing the foreign functional DNA sequence;

25. The method of Claim 24 wherein the oncogenic DNA is genomic DNA from a primary cell tumor or a tumor primary-cell line wherein such tumor or tumor cell-line has an origin corresponding to the primary cell type to be immortalized.

26.    The method of Claim 24 wherein the oncogene(s) is/are from a primary cell tumor or a tumor primary cell line wherein such tumor or tumor cell line has an origin corresponding to the primary cell type to be immortalized.

27.    The method of Claim 24 wherein the primary cells are of hematopoietic origin.

28.    The method of Claim 27 wherein the primary cells are multipotential stem cells, B-lymphocytes, B-lineage precursors, T-lympocytes, T-lineage precursors, myeloid stem cells, monocytes, macrophages, neutrophils, eosinophils, megakaryocytes or mast cells.

29.    The method of Claim 28 wherein the primary cells are B-lymphocytes.

30.    The method of Claim 29 wherein the B lymphocytes have been activated/stimulated with an antigen.

31.    The method of Claim 29 wherein the oncogene(s) is the Ha-ras oncogene or the p53-H7 oncogene.

32.    The method of Claim 29 wherein the oncogenic DNA is genomic DNA from BJAB.

33.    The method of Claim 29 wherein the oncogenic DNA is genomic DNA from the REH cell line.

34.    The method of Claim 29 wherein the oncogenic DNA is genomic DNA from the EL4 cell line.

35.    A cotransfected immortalized primary cell line produced by the method of Claim 24.

36.  The cell line of Claim 35 wherein the oncogenic DNA is genomic DNA from a primary cell tumor or a tumor primary-cell line wherein such tumor or tumor cell-line has an origin corresponding to the primary cell type to be immortalized.

37.  The cell line of Claim 35 wherein the oncogene(s) is/are from a primary cell tumor or a tumor primary cell line wherein such tumor or tumor cell line has an origin corresponding to the primary cell type to be immortalized.

38.  The cell line of Claim 35 wherein the primary cells are of hematopoietic origin.

39.  The cell line of Claim 38 wherein the primary cells are multipotential stem cells, B-lymphocytes, B-lineage precursors, T-lymphocytes, T-lineage precursors, myeloid stem cells, monocytes, macrophages, neutrophils, eosinophils, megakaryocytes or mast cells.

40.  The cell line of Claim 39 wherein the primary cells are B-lymphocytes.

41.  The cell line of Claim 40 wherein the B lymphocytes have been activated/stimulated with an antigen.

42.  The cell line of Claim 40 wherein the oncogene(s) is the Ha-ras oncogene.

43.  The cell line of Claim 40 wherein the oncogenic DNA is genomic DNA from BJAB.

44.  The cell line of Claim 40 wherein the oncogenic DNA is genomic DNA from the REH cell line.

45. The cell line of Claim 40 wherein the oncogenic DNA is genomic DNA from the EL4 cell line.

46. A method of producing biological products whose coding sequence is comprised by the DNA contained within a cotransfected immortalized primary cell line containing a foreign functional DNA sequence which comprises:

(a) providing normal, activated/stimulated primary cells;

(b) cotransfecting such primary cells with oncogenic DNA/oncogene(s) and a foreign functional DNA sequence (which, optionally, are comprised by liposomes) by electroporation thereby producing cotransfected primary cells;

(c) recovering from such cotransfected primary cells the cotransfected immortalized primary cell line containing the foreign functional DNA sequence; and

(d) culturing such cotransfected immortalized cell line under conditions which enable the production of such biological products.

47. An immortalized primary cell line provided that such immortalization is the result of transfection of a normal primary cell with DNA consisting essentially of oncogene(s).

48. The cell line of Claim 47 wherein the oncogene(s) is/are from a primary cell tumor or a tumor primary cell line wherein such tumor or tumor cell line has an origin corresponding to the primary cell type immortalized.

49. The cell line of Claim 47 wherein the primary cell is of hematopoietic origin.

50. The cell line of Claim 49 wherein the primary cell is a multipotential stem cell, B-lymphocyte, B-lineage precursor, T-lympocyte, T-lineage precursor, myeloid stem cell, monocyte, macrophage, neutrophil, eosinophil, megakaryocyte or mast cell.

51. The cell line of Claim 50 wherein the primary cell is a B-lymphocyte.

52. The cell line of Claim 51 wherein the oncogene(s) is the Ha-ras oncogene or the p53-H7 oncogene.

Claims for the Contracting States : AT, ES, GR

1. A method for producing an immortalized primary cell line which comprises :

(a) providing normal, activated/stimulated primary cells;

(b) transfecting such primary cells with oncogenic DNA/oncogene(s) (which, optionally, is comprised by liposomes) by electroporation thereby producing transfected primary cells; and

(c) recovering from such transfected primary cells an immortalized primary cell line.

2. The method of Claim 1 wherein the oncogenic DNA is genomic DNA from a primary cell tumor or a tumor primary-cell line wherein such tumor or tumor cell-line has an origin corresponding to the primary cell type to be immortalized.

3. The method of Claim 1 wherein the oncogene(s) is/are from a primary cell tumor or a tumor primary cell line wherein such tumor or tumor cell line has an origin corresponding to the primary cell type to be immortalized.

4. The method of Claim 2 wherein the primary cells are of hematopoietic origin.

5. The method of Claim 3 wherein the primary cells are multipotential stem cells, B-lymphocytes, B-lineage precursors, T-lymphocytes, T-lineage precursors, myeloid stem cells, monocytes, macrophages, neutrophils, eosinophils, megakaryocytes or mast cells.

6. The method of Claim 4 wherein the primary cells are B-lymphocytes.

7. The method of Claim 5 wherein the B-lymphocytes have been activated/stimulated with an antigen.

8. The method of Claim 5 wherein the oncogene(s) is the Ha-ras oncogene or the p53-H7 oncogene.

9. The method of Claim 6 wherein the oncogenic DNA is genomic DNA from BJAB.

10. The method of Claim 6 wherein the oncogenic DNA is genomic DNA from the REH cell line.

11. The method of Claim 6 wherein the oncogenic DNA is genomic DNA from the EL4 cell line.

12. A method of producing biological products whose coding sequence is comprised by the DNA contained within an immortalized primary cell line which comprises :

(a) providing normal, activated/stimulated primary cells.

(b) transfecting such primary cells with oncogenic DNA/oncogene(s) (which, optionally, is comprised by liposomes) by electroporation thereby producing transfected primary cells;

(c) recovering from such transfected primary cells the immortalized primary cell line; and

(d) culturing such immortalized cell line under conditions which enable the production of such biological products.

13. A method for production of a cotransfected immortalized primary cell line containing a foreign functional DNA sequence which comprises :

(a) providing normal, activated/stimulated primary cells;

(b) cotransfecting such primary cells with oncogenic DNA/oncogene(s) and the foreign functional DNA sequence (which, optionally, are comprised by liposomes) by electroporation thereby producing cotransfected primary cells, and

(c) recovering from such cotransfected primary cells an immortalized primary cell line containing the foreign functional DNA sequence;

14. The method of Claim 13 wherein the oncogenic DNA is genomic DNA from a primary cell tumor or a tumor primary-cell line wherein such tumor or tumor cell-line has an origin corresponding to the primary cell type to be immortalized.

15. The method of Claim 13 wherein the oncogene(s) is/are from a primary cell tumor or a tumor primary cell line wherein such tumor or tumor cell line has an origin corresponding to the primary cell type to be immortalized.

16. The method of Claim 13 wherein the primary cells are of hematopoietic origin.

17. The method of Claim 16 wherein the primary cells are multipotential stem cells, B-lymphocytes, B-lineage precursors, T-lymphocytes, T-lineage precursors, myeloid stem cells, monocytes, macrophages, neutrophils, eosinophils, megakaryocytes or mast cells.

18. The method of Claim 17 wherein the primary cells are B-lymphocytes.

19. The method of Claim 18 wherein the B lymphocytes have been activated/stimulated with an antigen.

20. The method of Claim 18 wherein the oncogene(s) is the Ha-ras oncogene or the p53-H7 oncogene.

21. The method of Claim 18 wherein the oncogenic DNA is genomic DNA from BJAB.

22. The method of Claim 18 wherein the oncogenic DNA is genomic DNA from the REH cell line.

23. The method of Claim 18 wherein the oncogenic DNA is genomic DNA from the EL4 cell line.

24. A method of producing biological products whose coding sequence is comprised by the DNA contained within a cotransfected immortalized primary cell line containing a foreign functional DNA sequence which comprises :

(a) providing normal, activated/stimulated primary cells;

(b) cotransfecting such primary cells with oncogenic DNA/oncogene(s) and a foreign functional DNA sequence (which, optionally, are comprised by liposomes) by electroporation thereby producing cotransfected primary cells;

(c) recovering from such cotransfected primary cells the cotransfected immortalized primary cell line containing the foreign functional DNA sequence; and

(d) culturing such cotransfected immortalized cell line under conditions which enable the production of such biological products.